# EUROPEAN PATENT SPECIFICATION

(11) **EP 1 674 867 B1**
(45) Date of publication and mention of the grant of the patent: **22.07.2009**
(21) Application number: 05077890.1
(22) Date of filing: 15.12.2005
(51) Int. Cl.: G01N 33/53, B01L 3/00

(54) **Method of rapid antigen extraction**
Verfahren zur raschen Antigenextraktion
Procédé d'extraction antigène rapide

(30) Priority: 21.12.2004 IT MI20042434
(43) Date of publication of application: 28.06.2006
(73) Proprietor: ABS Advanced Biomedical Systems Srl, 20063 Cernusco S/N (IT)
(72) Inventor: Giordano, Paolo, 20090 Segrate (MI) (IT)
(74) Representative: Long, Giorgio

(56) References cited:
- EP-A- 0 659 437
- WO-A-95/25948
- WO-A-05/071388
- US-A- 4 150 950
- US-A- 4 673 639
- US-A- 4 851 337
- US-A- 5 415 994
- US-A- 5 869 272
- US-B1- 6 180 395
- US-B1- 6 660 469
- PATENT ABSTRACTS OF JAPAN vol. 1995, no. 06, 31 July 1995 (1995-07-31) -& JP 07 059555 A (NIPPON HEALTH SCI KK; others: 01), 7 March 1995 (1995-03-07)

## Description

The present invention is about a method for preserving two or more reagents used in a chemical reaction.

Different methods for preserving two or more reagents, in order to put them in contact only when they have to be used, are known in the state of the art. For example, in pharmaceutical preparations, a reagent is commonly preserved in a solid state and it is dissolved in a liquid state solvent just before the use, by perforating a mechanical partition wall.

Other preservation methods have been described as well; in which two or more containers, each containing a reagent, are inserted into a single main container inside which, after the breakage of the internal containers, the reagent mixing takes place.

The traditional methods of extraction of saccharidic antigens from group A streptococcus provide for the use of liquid reagents (Lennett, E.H., Ed., Manual of Clinical Microbiology, Fourth Edition, American Society of Microbiology, Washington, D.C., 1985, pages 170-171). Typically, two liquid reagents are used in the extraction stage: an acid (acetic, hydrochloric or citric acid) and a sodium nitrite solution. The two reagents are mixed in a test tube in which the *wad* used to take the biological sample to be examined is inserted.

In other known methods, at least one of the two reagents is in a solid state (US Patent No. 5,536,646), or the reagents are contained in test tubes or flasks with several separate compartments, each containing a single reagent to be mixed immediately before use (US Patent No. 4,673,639).

By mixing the two reagents, nitrous acid, which is a relatively unstable acid, is produced, thus requiring that the reagents are mixed immediately before the extraction process starts. Otherwise, the instability of the resulting nitrous acid solution can reduce the extraction effectiveness. In fact, if the reagents are prematurely mixed with respect to the biological sample addition, according to what described in US Patent No. 4,851,337, the nitrous acid decomposition takes place and the extraction solution can lose its effectiveness in a very short time.

According to the method described in US Patent No. 5,415,994, the extraction takes place in a well directly obtained in the cartridge containing the immunochromatographic strip. One of the two reagents is contained in a flask, that contains in its turn a phial with the second reagent. The operator mixes the two reagents by breaking the phial and then pours the mixture in the well in which the swab is placed. In this way, the operator does not have to count the drop number of each reagent. However, in this case too the extraction takes place after the two reagents have been mixed. Moreover, the insertion of the swab in the well sometimes causes the even partial occlusion of the liquid drainage conduit, and thus the flow is slowed down or even blocked. Otherwise, if the swab is inserted in a manner such that the liquid does not flow therethrough before reaching the immunochromatographic chamber, the reaction liquid can reach the immunochromatographic membrane before the extraction takes place.

According to other known methods (US Patent No. 5,494,801), a third reagent is added to the two default ones in order to neutralize the solution before the chromatographic stage takes place. At present, however, the use of three reagents is considered too complicated for the operator, and thus the systems providing for the use of two reagents only are preferred. Furthermore, even these methods do not avoid the risk of effectiveness reduction of the extraction due to the time elapsed between the reagent mixing and the sample insertion.

Methods that provide for the insertion of the sample, on which the extraction has to be performed, in a device before adding the reagents have been described too (US Patent No. 6,168,956). In these methods, the operator must apply the reagents according to the optimal time sequence. However, the need to determine the volume of the reagents (for example, by counting the reagent drops) still remains, as well as the possibility to use the same reagent twice.

The traditional methods of extraction of lipopolysaccharidic antigens from Chlamydia trachomatis provide for the use of an alkaline reagent able to extract the lipopolysaccharidic antigens, in which an acid or a buffer is inserted to neutralize the extraction. According to a traditional method, the alkaline reagent consists of sodium hydroxide and the acid reagent for neutralizing the extraction solution is hydrochloric acid.

The swab through which the biological sample has been taken is inserted into a test tube containing the alkaline reagent for the extraction and it is agitated for a predetermined time, after which the neutralization reagent is added.

PCT application WO 95/25948, US Patent 6,180,395, Japanese patent application JP 07059555 and PCT application WO 2005/071388 all describe multi-compartment sample collecting and assay devices. However, none of these cited prior art documents discloses or suggests a method of extracting antigens as claimed in the appended claims.

It is thus an object of the present invention to overcome the problems of the aforementioned prior art method and devices.

The present invention can be applied in all the situations in which two or more reagents, that can start a chemical reaction together with a sample to be treated, can not be mixed together before adding the sample. For example, it is possible that the sample has to be put in contact with an unstable reaction product, or with more than one reagent, in a predetermined sequence.

More specfically, the present invention can be applied to the bacterial antigens extraction processes performed with swabs on human or animal samples. In particular, the present invention can be used for the extraction of saccharidic antigens from group A and B streptococcus, as well as of lipopolysaccharidic antigens from Chlamydia.

The main object of the method of rapid antigen extraction according to the present invention is thus to simplify the aforementioned prior art extraction processes, in particular:
a) the operator does not have to predetermine and check the reagent volume, for example by counting the dispensed reagent drops;
b) the time needed to dissolve one or more solid state reagents is not required;
c) the time elapsed between the reagent mixing and the insertion of the swab with the biological sample does not reduce the extraction effectiveness, for example when the highly unstable nitrous acid is used.

Further characteristics and advantages of the method of rapid antigen extraction according to the present invention, and the device for performing said method, will be better highlighted in the following description of preferred embodiments, given in an explanatory but not limiting way with reference to the annexed figures of drawings, in which:
Figure 1 is a schematic sectional view of a first embodiment of a device for performing the method of rapid antigen extraction according to the present invention;
Figure 2 is a schematic sectional view of a device for performing an alternative method of rapid antigen extraction; and
Figure 3 is a schematic sectional view of an embodiment of a flexible extraction device inserted in a rigid tube for the breakage of the mechanical barrier between the two reagents.

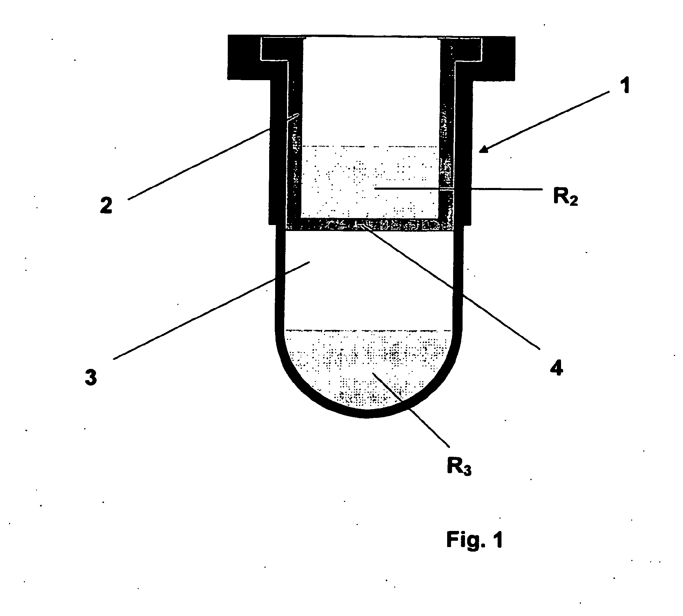

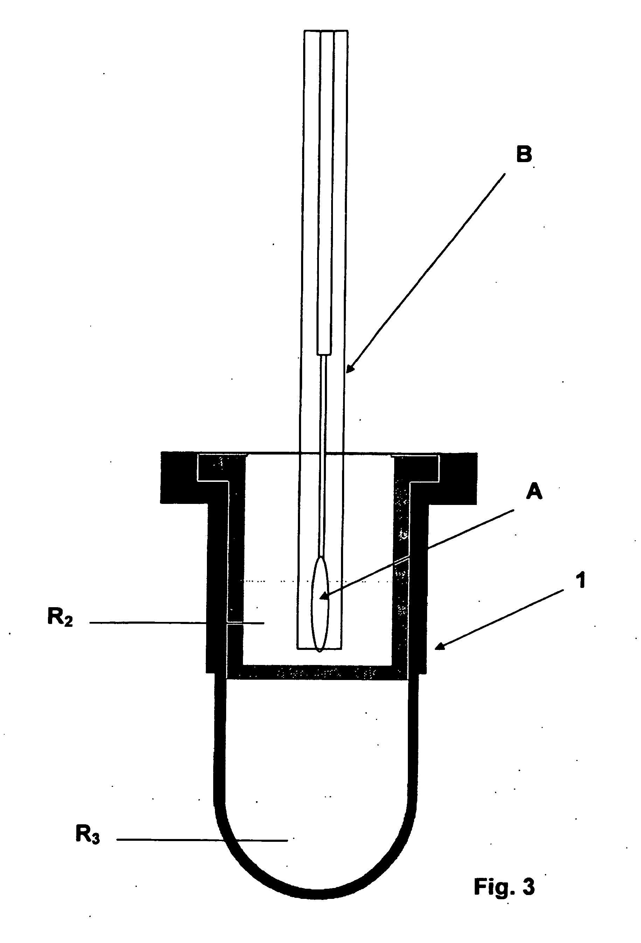

With reference to Figure **1****,** according to the method of the invention, the two reagents are poured in predetermined volumes in a preferably but not necessarily cylindrical test tube **1** during the manufacturing stage of the device for performing said method, said test tube **1** comprising a first inner container **2** able to be inserted in a second outer container **3** that forms the main body of the test tube **1.** The container **2** is configurated to form a cap for the container **3.** The two containers **2** and **3** are then assembled so that the swab **A** (Figure 3) with the sample to be tested sequentially passes through the reagent **R₂,** placed inside the container **2,** and the reagent **R₃,** placed inside the container **3,** respectively by breaking the mechanical barrier **4** that forms the bottom wall of said container **2** and separates said two reagents **R₂** and **R₃.** The containers **2** and **3** can be manufactured with any kind of material compatible with the reagents **R₂** and **R₃** contained therein, and can have a proper shape and a sufficient volume to contain the swab **A,** said reagents **R₂** and **R₃** being in turn either in liquid or in solid state. The test tube **1** is sealed at its top using any known sealing system, for example with a metallic sheet (not shown).

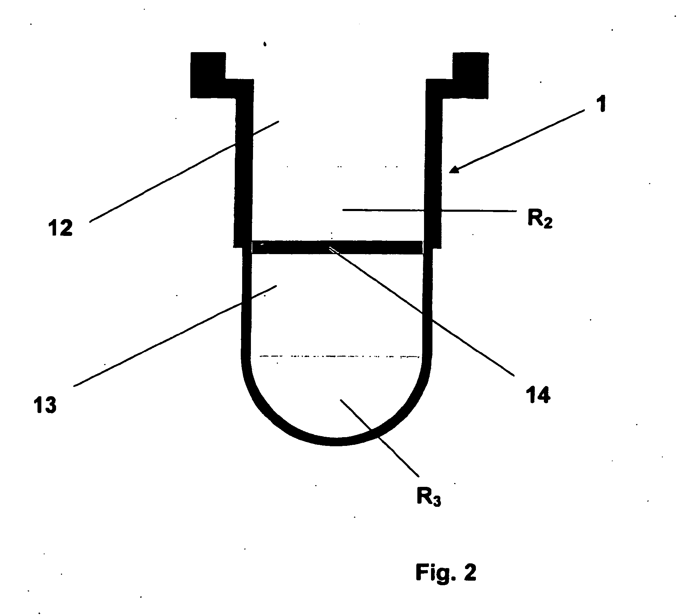

A device for performing alternative method is shown in Figure **2****.** After pouring the reagent **R₃** in the test tube **1,** it is possible to form a partition wall **14** in said test tube **1** having the same function of the container **2** mechanical barrier **4,** for example by adding solid paraffin, heating it up to its melting and thus letting it cool down until it forms a proper physical partition element **14** similar to said mechanical barrier **4,** thus being able to define two separate containers, an upper one **12** and a lower one **13,** inside the same test tube **1.** At this point it is possible to add the second reagent **R₂** into the so formed upper container **12** of the test tube **1.** In this case too the test tube **1** is subsequently sealed at its top using a known sealing system.

The device consisting of the test tube **1** for performing the method according to the invention is thus able to ensure that the two reagents **R₂** and **R₃** are put in contact only when the swab **A** bearing the sample is present. Therefore, according to the method of the invention, the transportation of the first reagent to the second reagent is performed by the swab **A** itself.

According to a preferred aspect of the invention, the sample is taken with a pharyngeal swab **A** following well known procedures. The swab **A** is then inserted into the first container **2** or **12** of the test tube **1**, after the removal of the seal, and it is then driven in the second container **3** or **13,** by breaking the barrier **4** between the containers **2** and **3** or the partition wall **14** between the containers **12** and **13.** The extraction of the antigen by means of the so formed nitrous acid is thus started. When the expected extraction time is lapsed, the swab **A** is removed, preferably with the first container **2** if present, from the test tube **1** and the liquid can be poured directly from said test tube **1** into the immunochromatographic device for the antigen detection.

For example, according to the method of the invention, the reagent **R₂** contained in the first container **2, 12** can be a 0,4 M acetic acid. The operator inserts the swab **A** into the first container **2, 12** of the test tube **1.** The reagent **R₂** is almost fully absorbed by the swab **A.** By pushing the swab **A** against the bottom **4, 14** of the container **2, 12,** said bottom **4, 14** breaks, allowing said swab **A** to pass through and thus to reach the reagent **R₃,** for instance a 2 **M** sodium nitrite, in the second container **3, 13.** At this point, the nitrous acid formation reaction takes place, due to the antigen presence on the swab **A.** Therefore, the antigen extraction takes place in the best conditions for the effectiveness of said extraction. Once the expected time for the extraction of the bacterial antigens from the swab **A** is lapsed, the swab **A** is removed from the test tube **1** and the liquid can be poured in the immunochromatographic strip cartridge well.

Another example of the method according to the present invention allows to extract Chlamydia antigens from cervical or urethral swabs. The sample is taken with a cervical or urethral swab according to well known procedures. The swab is then inserted into the first container **2, 12** of the test tube **1,** after the removal of said test tube seal, and it is left in contact with the reagent **R₂** for the required extraction time. Once the extraction is finished, the swab is pushed into the second container **3** by breaking the barrier **4,** or into the second container **13** by breaking the partition wall **14,** and it is put in contact with the neutralization reagent **R₃.**

In this case, the reagents comprise an alkaline reagent **(R₂)** and an acidic neutralization reagent **(R₃).**

According to a traditional method, the cervical or urethral swab is inserted into a test tube containing 5 drops of 0,2 N sodium hydroxide, and it is left in the solution for 2 minutes. After shaking the swab, a predetermined volume of 0,1 N hydrochloric acid is added to neutralize the extraction solution. After shaking the swab again, said swab is then removed and a certain volume of the extraction solution is added to the test cartridge.

In order to take biological samples from particular sites, for example from the nasal cavities or the urethra, devices having a flexible and thin structure are available on the market, thus being difficult to break the partition wall between the two reagents with said devices. In this case, the sampling device can be inserted in advance into an assembly provided with the proper stiffness and resistance features for breaking the partition wall. For example, after the insertion of the sampling swab **A** into the test tube **1,** it is possible to surround said swab **A** with a tube **B** having a proper diameter. By pushing the tube **B,** that breaks the barrier **4,** the swab **A** is transported into the container **3, 13** (see Figure 3).

It should be understood that several modifications could be made to the device, formed by the test tube **1,** that performs the method of rapid antigen extraction extraction according to the present invention, as it is also defined in the appended claims. For example, the sealing of the test tube **1** can be obtained by using a cap, or by thermal sealing with an aluminium sheet coupled with polyethylene. Furthermore, although in the description the sample collection system is indicated as a "swab", this denomination is merely used for convenience, since the most common systems for taking the A group streptococcus are the pharyngeal swabs, while the most common systems for taking the Chlamydia trachomatis are the cervical or urethral swabs. Therefore, it should be obvious for a man skilled in the art that it is possible to use any swab compatible with the immunological array format.

## Claims

1. A method for extracting antigens to be analysed by immunochromatographic methodology comprising the following steps:
inserting a swab (A), through which a chemical substance sample to be examined has been taken, into a first container (2, 12) belonging to a test tube (1) and
containing a first reagent (R2), said swab (A) carrying said chemical substance sample being able to at least partially absorb said first reagent (R2) within a predetermined time period;
exerting a pressure with said swab (A), after the at least partially absorption of said first reagent (R2) by said swab (A) carrying said chemical substance sample, against the bottom wall (4, 14) of said first container (2, 12), causing said swab (A) to break said bottom wall (4, 14) and to pass through said bottom wall (4, 14);
bringing said swab (A), carrying said chemical substance sample and at least a part of said first reagent (R2), in contact with at least a second reagent (R3) contained into a second container (3, 13) belonging to said test tube (1), ensuring that said first reagent (R2) and said second reagent (R3) are put in contact one with another and with said chemical substance sample only when said_swab (A) carrying said chemical substance sample to be examined is present;
removing said swab (A) from said second container (3, 13) belonging to said test tube (1) after a predetermined time period and once the reaction between said first reagent (R2), said second reagent (R3) and said chemical substance sample is completed,
**characterized in that**
said swab (A) as well as said first container (2) together with its perforated bottom wall (4), can be removed from said test tube (1).

2. The method according to claim 1, **characterized in that** said first reagent (R2) and said second reagent (R3) are poured in predetermined volumes into said first container (2, 12) and into said second container (3, 13) respectively during the manufacturing stage of said test tube (1).

3. The method according to claim 1, **characterized in that** said swab (A) sequentially passes through said first reagent (R2) and said second reagent (R3).

4. The method according to claim 1, **characterized in that** said first reagent (R2) and said second reagent (R3) are able to form nitrous acid.

5. The method according to claim 1, **characterized in that** said second reagent (R3) is in a solid state.

6. The method according, to claim 1, **characterized in that** said swab (A) is transported through said bottom wall (4) by means of a rigid device (B) able to break said bottom wall (4).

7. The method according to claim 1, **characterized in that** said chemical substance sample to be examined must remain into said first reagent (R2) for a predetermined time period before being transferred into said second reagent (R3).

8. The method according to claim 7, **characterized in that** said first reagent (R2) and said second reagent (R3) are a base and a neutralization solution respectively.

9. The method according to claim 7, **characterized in that** said first reagent (R2) and said second reagent (R3) are an acid and a neutralization solution respectively.

10. Use of the method according to claim 4 for the extraction of saccharidic antigens from group A and group B streptococcus, taken with a swab (A).

11. Use of the method according to claims 8 or 9 for the extraction of lipopolysaccharidic antigens from Chlamydia trachomatis, taken with a swab (A).

12. Use of the method according to one or more of claims 1 to 9 for the extraction of saccharidic antigens from group A and group B streptococcus and lipopolysaccharidic antigens from Chlamydia trachomatis, taken with a swab (A).

## Patentansprüche

1. Ein Verfahren zum Extrahieren von Antigenen, die anhand einer immunochromatographischen Methodologie analysiert werden sollen, wobei das Verfahren folgende Schritte umfasst:
Einführen eines Tupfers (A), durch den eine Probe einer chemischen Substanz, die untersucht werden soll, genommen wurde, in einen ersten Behälter (2, 12), der zu einem Reagenzglas (1) gehört und ein erstes Reagens (R2) enthält, wobei der Tupfer (A), der die Probe der chemischen Substanz trägt, in der Lage ist, das erste Reagens (R2) innerhalb eines vorbestimmten Zeitraums zumindest teilweise zu absorbieren;
Ausüben eines Drucks mit dem Tupfer (A), nach der zumindest teilweisen Absorption des ersten Reagens (R2) durch den Tupfer (A), der die Probe der chemischen Substanz trägt, gegen die untere Wandung (4, 14) des ersten Behälters (2, 12), wodurch bewirkt wird, dass der Tupfer (A) die untere Wandung (4, 14) durchbricht und die untere Wandung (4, 14) passiert; Inkontaktbringen des Tupfers (A), der die Probe der chemischen Substanz und zumindest einen Teil des ersten Reagens (R2) trägt, mit zumindest einem zweiten Reagens (R3), das in einem zweiten Behälter (3, 13), der zu dem Reagenzglas (1) gehört, enthalten ist, wodurch gewährleistet wird, dass das erste Reagens (R2) und das zweite Reagens (R3) nur dann miteinander und mit der Probe der chemischen Substanz in Kontakt kommen, wenn der Tupfer (A), der die Probe der chemischen Substanz trägt, die untersucht werden soll, vorliegt;
Entnehmen des Tupfers (A) aus dem zweiten Behälter (3, 13), der zu dem Reagenzglas (1) gehört, nach einem vorbestimmten Zeitraum und nachdem die Reaktion zwischen dem ersten Reagens (R2), dem zweiten Reagens (R3) und der Probe der chemischen Substanz abgeschlossen ist,
**dadurch gekennzeichnet, dass**
der Tupfer (A) sowie der erste Behälter (2) zusammen mit dessen perforierter unterer Wandung (4) aus dem Reagenzglas (1) entnommen werden können.

2. Das Verfahren gemäß Anspruch 1, **dadurch gekennzeichnet, dass** das erste Reagens (R2) und das zweite Reagens (R3) während der Herstellungsstufe des Reagenzglases (1) in vorbestimmten Volumina in den ersten Behälter (2, 12) beziehungsweise in den zweiten Behälter (3, 13) geschüttet werden.

3. Das Verfahren gemäß Anspruch 1, **dadurch gekennzeichnet, dass** der Tupfer (A) nacheinander das erste Reagens (R2) und das zweite Reagens (R3) passiert.

4. Das Verfahren gemäß Anspruch 1, **dadurch gekennzeichnet, dass** das erste Reagens (R2) und das zweite Reagens (R3) in der Lage sind, salpetrige Säure zu bilden.

5. Das Verfahren gemäß Anspruch 1, **dadurch gekennzeichnet, dass** das zweite Reagens (R3) in einem festen Zustand vorliegt.

6. Das Verfahren gemäß Anspruch 1, **dadurch gekennzeichnet, dass** der Tupfer (A) anhand einer starren Vorrichtung (B), die in der Lage ist, die untere Wandung (4) zu durchbrechen, durch die untere Wandung (4) hindurch transportiert wird.

7. Das Verfahren gemäß Anspruch 1, **dadurch gekennzeichnet, dass** die Probe der chemischen Substanz, die untersucht werden soll, einen vorbestimmten Zeitraum lang in dem ersten Reagens (R2) verbleiben muss, bevor sie in das zweite Reagens (R3) transferiert wird.

8. Das Verfahren gemäß Anspruch 7, **dadurch gekennzeichnet, dass** das erste Reagens (R2) und das zweite Reagens (R3) eine Base beziehungsweise eine Neutralisationslösung sind.

9. Das Verfahren gemäß Anspruch 7, **dadurch gekennzeichnet, dass** das erste Reagens (R2) und das zweite Reagens (R3) eine Säure beziehungsweise eine Neutralisationslösung sind.

10. Verwendung des Verfahrens gemäß Anspruch 4 zur Extraktion von saccharidischen Antigenen aus Streptokokken der Gruppe A und der Gruppe B, die mit einem Tupfer (A) entnommen werden.

11. Verwendung des Verfahrens gemäß Anspruch 8 oder 9 zur Extraktion von lipopolysaccharidischen Antigenen aus Chlamydia trachomatis, die mit einem Tupfer (A) entnommen werden.

12. Verwendung des Verfahrens gemäß einem oder mehreren der Ansprüche 1 bis 9 zur Extraktion von saccharidischen Antigenen aus Streptokokken der Gruppe A und der Gruppe B und von lipopolysaccharidischen Antigenen aus Chlamydia trachomatis, die mit einem Tupfer (A) entnommen werden.

## Revendications

1. Procédé d'extraction d'antigènes destinés à être analysés par une méthodologie immunochromatographique comprenant les étapes suivantes :
insertion d'un écouvillon (A), par l'intermédiaire duquel un échantillon de substance chimique destiné à être examiné a été prélevé, dans un premier récipient (2, 12) appartenant à un tube à essais (1) et contenant un premier réactif (R2), ledit écouvillon (A) portant ledit échantillon de substance chimique étant apte à absorber au moins partiellement ledit premier réactif (R2) dans une période de temps prédéterminée ;
exercice d'une pression avec ledit écouvillon (A), après l'absorption au moins partielle dudit premier réactif (R2) par ledit écouvillon (A) portant ledit échantillon de substance chimique, contre une paroi de fond (4, 14) dudit premier récipient (2, 12), faisant que ledit écouvillon (A) casse ladite paroi de fond (4, 14) et passe à travers ladite paroi de fond (4, 14) ;
la mise dudit écouvillon (A), portant ledit échantillon de substance chimique et au moins une partie dudit premier réactif (R2), en contact avec au moins un deuxième réactif (R3) contenu dans un deuxième récipient (3, 13) appartenant audit tube à essais (1), assurant que ledit premier réactif (R2) et ledit deuxième réactif (R3) ne sont mis en contact l'un avec l'autre et avec ledit échantillon de substance chimique que lorsque ledit écouvillon (A) portant ledit échantillon de substance chimique destiné à être examiné est présent ;
enlèvement dudit écouvillon (A) dudit deuxième récipient (3, 13) appartenant audit tube à essais (1) après une période de temps prédéterminée et une fois que la réaction entre ledit premier réactif (R2), ledit deuxième réactif (R3) et ledit échantillon de substance chimique est terminée,
**caractérisé en ce que**
ledit écouvillon (A) ainsi que ledit premier récipient (2) avec sa paroi de fond (4) perforée peuvent être enlevés dudit tube à essais (1).

2. Procédé selon la revendication 1, **caractérisé en ce que** ledit premier réactif (R2) et ledit deuxième réactif (R3) sont versés dans des volumes prédéterminés à l'intérieur dudit premier récipient (2, 12) et à l'intérieur dudit deuxième récipient (3, 13) respectivement pendant l'étape de fabrication dudit tube à essais (1).

3. Procédé selon la revendication 1, **caractérisé en ce que** ledit écouvillon (A) passe séquentiellement à travers ledit premier réactif (R2) et ledit deuxième réactif (R3).

4. Procédé selon la revendication 1, **caractérisé en ce que** ledit premier réactif (R2) et ledit deuxième réactif (R3) sont aptes à former de l'acide nitreux.

5. Procédé selon la revendication 1, **caractérisé en ce que** ledit deuxième réactif (R3) est à l'état solide.

6. Procédé selon la revendication 1, **caractérisé en ce que** ledit écouvillon (A) est transporté à travers ladite paroi de fond (4) au moyen d'un dispositif rigide (B) apte à casser ladite paroi de fond (4).

7. Procédé selon la revendication 1, **caractérisé en ce que** ledit échantillon de substance chimique destiné à être examiné doit rester dans ledit premier réactif (R2) pendant une période de temps prédéterminée avant d'être transféré dans ledit deuxième réactif (R3).

8. Procédé selon la revendication 7, **caractérisé en ce que** ledit premier réactif (R2) et ledit deuxième réactif (R3) sont une base et une solution de neutralisation respectivement.

9. Procédé selon la revendication 7, **caractérisé en ce que** ledit premier réactif (R2) et ledit deuxième réactif (R3) sont un acide et une solution de neutralisation respectivement.

10. Utilisation du procédé selon la revendication 4 pour l'extraction d'antigènes saccharidiques de streptocoques de Groupe A et de Groupe B, prélevés avec un écouvillon (A).

11. Utilisation du procédé selon les revendications 8 ou 9 pour l'extraction d'antigènes lipopolysaccharidiques de *Chlamydia trachomatis*, prélevés avec un écouvillon (A).

12. Utilisation du procédé selon une ou plusieurs des revendications 1 à 9 pour l'extraction d'antigènes saccharidiques de streptocoques de Groupe A et de Groupe B et d'antigènes lipopolysaccharidiques de *Chlamydia trachomatis*, prélevés avec un écouvillon (A).
